# EUROPEAN PATENT APPLICATION

(11) **EP 1 530 910 A2**
(43) Date of publication of application: **18.05.2005**
(21) Application number: 04105396.8
(22) Date of filing: 29.10.2004
(51) Int. Cl.: A23L 3/375, A23L 3/3481, A23L 3/358, A23L 3/3454, A23B 4/09, A23B 4/30, A61L 2/18, A61L 2/00

(54) **Cold shock method improvements**

(30) Priority: 12.11.2003 US 519679 P; 03.09.2004 US 934010
(71) Applicant: L'air liquide, Société anonyme à Directoire et Conseil de Surveillance pour l'Etude et l'Exploitation des Procédés G. Claude, 75321 Paris Cedex 07 (FR)
(72) Inventor: Giacobbe, Frederick W., 60540, Napervielle IL (US); Yuan, James T.C., 60565, Napervielle IL (US)
(74) Representative: Mellul-Bendelac, Sylvie Lisette

(57) **Abstract**

The invention is a method of cooling objects by spraying with a fluid to obtain a desired cooling effect. The fluid may be sprayed directly or indirectly on the objects. The fluid may be liquid nitrogen, liquid argon, liquid oxygen, liquid carbon dioxide, or any combination thereof. The fluid may include an additive that helps sanitize the object. This additive may be an oxidizing agent such as ozone, chlorine, chlorine compounds, hydrogen peroxide solutions, oxonia solutions, or combinations thereof. The regulation may be by way of time or flow. The regulation may involve equal time intervals of spraying or unequal time intervals of spraying. The desired cooling effect may be to shock the surface microorganisms to make them susceptible to concurrent or subsequent treatments. The desired cooling effect may be to promote the destruction or inactivation of surface microorganisms without cooling or freezing the object throughout.

## Description

### CROSS REFERENCE TO RELATED APPLICATIONS

This application claims the benefit of U.S. Provisional Application No. 60/519,679, filed November 12, 2003, the entire contents of which are incorporated herein by reference.

### BACKGROUND

It is well known that chilling food products reduces bacterial growth and retards the onset of spoilage, thereby increasing shelf life. However, in the past 20 years, it has become known that some microorganisms have been able to adapt to a period of such chilled, low growth temperature. In response to this ability to adapt, recent investigations into the cold shock responses of various microorganisms have been conducted. It has been found that cold shocking can introduce stresses affecting biological structures, which can fatally injure the microorganism, or weaken the microorganism and make it more susceptible to antibacterial or disinfecting agents. Various difficulties have been associated with the attempts that have been made so far to try to rapidly cool food and thereby effectively introduce such a cold shock procedure into food processing.

One attempt at using cryogenic liquids for cold shocking a food product is described in U.S. Pat. No. 4,325,221, where animal carcasses are sprayed with liquid nitrogen. This disclosure teaches that the surface membranes of the carcass are to be rapidly frozen in order to seal in the loss of moisture from the carcass. The principle desired effect is to reduce moisture loss from the product.

Another attempt is described in U.S. Pat. No. 4,367,630, where carcasses are immersed into a tank of cryogenic fluid to crust-freeze the exterior Again, the desired effect is to reduce valuable weight loss of the carcass.

Yet another attempt is described in U.S. Pat. No. 4,940,599, where the surface of fresh meat is subjected to a cryogenic liquid just long enough to form a layer of ice, but not long enough to freeze the outermost, and innermost, layers of flesh on the carcass. The desired effect is to reduce spoilage in the appearance of the outermost layer of the flesh.

Another attempt is described in U.S. Pat. No. 5,471,846, where a mass of food product is introduced into a storage compartment, then into which an amount of cryogenic liquid is introduced, as a function of the weight of the food product present. The desired effect is to increase the overall efficiency of the chilling process using cryogenic liquids.

Another attempt is described in U.S. Pat. No. 5,577,392, where a cryogenic chiller is disclosed that uses a vortical flow pattern. The desired effect is to sweep any liquid or solid cryogen from the bottom surface of the tunnel and recirculate it around the food product.

There is also a need to disinfect and sanitize non-food objects, such as surgical, dental or laboratory instruments, prosthetic joints, dentures and similar objects. Traditional processes for sterilizing such objects have utilized steam, ethylene oxide, ionizing radiation, formaldehyde, and hot air. Within the past decade, new processes have been introduced that include peracetic acid, chlorine dioxide, plasmas, and ultva-violet light.

Each of these references suffers from the disadvantage that either the germs on the very surface, or just below the surface, of the object are not actually cold shocked. There is no control of the application of the cryogenic liquid to obtain the desired effect of cold shocking the surface pathogens. There is no additive introduced into the cryogenic liquid.

For the foregoing reasons, there is a need for a method for cold shocking food that allows control of the spraying to obtain a specified desired cooling effect. It would be an advance in the art of disinfection to couple this resulting cold shock effect with either the prior, concurrent, or subsequent application of an additive within the cryogenic fluid. There is a need in the industry for such an additive to further assist in the sanitization process.

### SUMMARY

The present invention is directed to a method that satisfies the need in society in general for a method for cold shocking food, or non-food objects, that allows the spraying of a fluid with an additive, and the regulation of this spraying to obtain a desired cooling effect. The present invention is also directed to a method that satisfies the need to combine this resulting cold shock effect with either the prior, concurrent, or subsequent application of an additive within the cryogenic fluid, wherein this additive is preferably an aid to sanitization.

This method comprises a method of cooling objects spraying this object with a fluid, where the fluid has an additive, and where the spraying is regulated to obtain a desired cooling effect. These objects may be either food or non-food objects. The fluid may be sprayed directly or indirectly on the objects. The fluid may be liquid nitrogen, liquid argon, liquid oxygen, liquid carbon dioxide, or any combination thereof.

This additive may be something that participates in the desired sanitization process of the object. This additive may be added prior to, coincident with, or subsequent to the actual cooling treatment. This additive may be an oxidizing agent such as ozone, chlorine, chlorine compounds, hydrogen peroxide solutions, oxonia solutions, or combinations thereof.

Oxonia is know to one skilled in the art to be a solution composed of either hydrogen peroxide and peristaltic acid, or hydrogen peroxide and peroxyacetic acid.

The regulation may be by way of time regulation or flow regulation. The regulation may involve equal time intervals of spraying or unequal time intervals of spraying. These spraying intervals may be interrupted by either equal time intervals or unequal time intervals of non-spraying.

The desired cooling effect is to shock the surface microorganisms to make them susceptible to concurrent or subsequent treatments. The desired cooling effect is to promote the destruction and inactivation of surface microorganisms either without cooling or freezing the object throughout, or while cooling or freezing the object throughout.

These and other features, aspects, and advantages of the present invention will become better understood with reference to the following description and appended claims.

### DESCRIPTION

Illustrative embodiments of the invention are described below. While the invention is susceptible to various modifications and alternative forms, specific embodiments thereof are herein described in detail. It should be understood, however, that the description herein of specific embodiments is not intended to limit the invention to the particular forms disclosed, but on the contrary, the intention is to cover all modifications, equivalents, and alternatives falling within the spirit and scope of the invention as defined by the appended claims.

It will of course be appreciated that in the development of any such actual embodiment, numerous implementation-specific decisions must be made to achieve the developer's specific goals, such as compliance with system-related and business-related constraints, which will vary from one implementation to another. Moreover, it will be appreciated that such a development effort might be complex and time-consuming, but would nevertheless be a routine undertaking for those of ordinary skill in the art having the benefit of this disclosure.

The present invention relates to a cryogenic rapid chilling process in which a mixture of fluid and an additive is used to cold shock an object. In one embodiment of the invention, the liquid cryogen and additive are mixed prior to use in the chilling process. In this way, both the liquid cryogen and the additive may be directed to the object simultaneously.

In another embodiment of the invention, the additive may be an ozidizing agent such as ozone, chlorine, chlorine compounds, hydrogen peroxide solutions, oxonia solutions, or combinations thereof.

In another embodiment of the invention, the additive may be an organic substance, an inorganic substance, a combination of two (or more) organic substances, a combination of two (or more) inorganic substances, or a combination of one (or more) organic and one (or more) inorganic substance.

In another embodiment of the invention, the regulation comprises the continuous (or semi-continuous) real-time regulation and adjustment of the additive (or additives) into a continuous spraying process, where the flowrate of the cryogenic fluid may either remain substantially constant or may vary over time.

In another embodiment of the invention, the liquid cryogen and the additive are introduced into the chilling process by two, or more, separate nozzles. In this way, the additive may be directed to the object prior to, simultaneous with, or subsequent to the chilling process.

In another embodiment of the invention, the additive may be directed to the object at a combination of prior to, simultaneous with, or subsequent to the chilling process, as desired by the operator. In another embodiment of the invention, different additives may be directed to the object at differing times in the chilling process.

In another embodiment of the invention, the object is sprayed with a first additive, and then sprayed with one (or more) additional additives. The first additive and one (or more) additional additives may be the same or different. They may be sprayed on simultaneously or consecutively.

In another embodiment of the invention, the flowrate of the additive may vary with time as a function of the progress of the chilling, or cold shock process. In another embodiment of the invention, the additive may be directed to certain discrete locations on the surface of the object to be chilled.

Food products, or non-food products, are rapidly cooled by the use of time and/or flow regulated spraying of liquefied cryogenic gases [e.g. liquid Nitrogen, liquid Argon, liquid Oxygen, liquid Carbon Dioxide (which may turn into the solid form during the spraying process instead of directly into the gas phase), and the like] directly or indirectly onto the object to be cooled.

Time regulated spraying means that the spraying process is deliberately designed to vary intermittently with time. In other words, the spraying process may vary at regulated and well controlled equal or unequal time intervals separated by equal or unequal time intervals of "non-spraying". These on/off time intervals may vary from the range of microseconds (or lower) up to minutes (or higher) in length until the desired cooling effect within the object so cooled has been achieved.

Flow regulated spraying means that the volumetric flow rate of the cooling fluid may also be deliberately varied with time during the equal or unequal spraying time intervals.

In one embodiment of the invention, the regulation of spraying intervals and/or non-spraying intervals, either by time or by flow rate, may coincide with the timing of the device that is introducing the objects to the cooling process. This regulation may be accomplished by way of sensors, or weighing devices, or other techniques commonly known in the industry.

In one embodiment of this invention, the regulation of spraying intervals and/or non-spraying intervals, either by time or by flow rate, may be accomplished by way of sensors located on, or within the body of, the object to be cooled.

In another embodiment, the object to be chilled is subsequently introduced to a chilling room, or a freezing room.
The invention is not limited to the preferred embodiments described above, but rather defined by the claims set forth below.

## Claims

1. A method of rapidly cooling objects comprising:
spraying said object with a fluid, wherein said fluid further comprises an additive, wherein said spraying is regulated to obtain a desired cooling effect.

2. The method of claim 1, wherein said objects are food objects.

3. The method of claim 1, wherein said objects are non-food objects.

4. The method of claims 1 to 3, wherein said object is sprayed indirectly with said fluid.

5. The method of claims 1 to 3, wherein said object is sprayed directly with said fluid.

6. The method of claims 1 to 5, wherein said fluid is selected from the group consisting of liquid nitrogen, liquid argon, liquid oxygen, liquid carbon dioxide, and combinations thereof.

7. The method of claims 1 to 6, wherein the additive is mixed with the fluid prior to use in the chilling process.

8. The method of claims 1 to 7, wherein said additive participates in a sanitization process.

9. The method of claims 1 to 8, wherein said sanitization process occurs before the cooling treatment.

10. The method of claims 1 to 8, wherein said sanitization process occurs during the cooling treatment.

11. The method of claims 1 to 8, wherein said sanitization process occurs after the cooling treatment.

12. The method of claims 1 to 7, wherein said additive is an oxidizing agent selected from the group consisting of ozone, chlorine, chlorine compounds, hydrogen peroxide solutions, oxonia solutions, or combinations thereof.

13. The method of claims 1 to 7, wherein said additive is selected from the group consisting of organic substances, inorganic substances, a combination of two or more organic substances, a combination of two or more inorganic substances, and a combination of one or more organic substance and one or more inorganic substance.

14. The method of claims 1 to 13, wherein said regulation is time regulation, wherein said spraying process varies intermittently with time.

15. The method of claims 1 to 14, wherein said time regulation involves equal time intervals of spraying.

16. The method of claims 1 to 14, wherein said time regulation involves unequal time intervals of spraying.

17. The method of claims 1 to 16, wherein said time regulation involves equal time intervals of non-spraying.

18. The method of claims 1 to 16, wherein said time regulation involves unequal time intervals of non-spraying.

19. The method of claims 1 to 18, wherein said regulation is flow regulation, wherein the volumetric flow rate during said spraying process varies with time.

20. The method of claims 1 to 19, wherein said regulation comprises the continuous regulation of said additives into a continuous spraying process, wherein the flow rate of the fluid remains substantially constant.

21. The method of claims 1 to 19, wherein said regulation comprises the continuous regulation of said additives into a semi-continuous spraying process, wherein the flow rate of the fluid remains substantially constant

22. The method of claims 1 to 19, wherein said flow regulation involves equal time intervals of spraying.

23. The method of claims 1 to 19, wherein said flow regulation involves unequal time intervals of spraying.

24. The method of claims 1 to 23, wherein said flow regulation involves equal time intervals of non-spraying.

25. The method of claims 1 to 23, wherein said flow regulation involves unequal time intervals of non-spraying.

26. The method of claims 1 to 20, wherein said time regulation involves equal time intervals of spraying.

27. The method of claims 1 to 20, wherein said time regulation involves unequal time intervals of spraying.

28. The method of claims 1 to 20, wherein said time regulation involves equal time intervals of non-spraying.

29. The method of claims 1 to 20, wherein said time regulation involves unequal time intervals of non-spraying.

30. The method of claims 1 to 29, wherein said desired cooling effect is to shock surface microorganisms to make them susceptible to a concurrent treatment.

31. The method of claims 1 to 30, wherein said desired cooling effect is to shock surface microorganisms to make them susceptible to a subsequent treatment.

32. The method of claims 1 to 31, wherein said desired cooling effect is to promote the destruction and inactivation of surface microorganisms without freezing the object throughout.

33. The method of claims 1 to 32, wherein said desired cooling effect is to promote the destruction and inactivation of surface microorganisms without cooling the object throughout.

34. The method of claims 1 to 33, wherein said desired cooling effect is to promote the destruction and inactivation of surface microorganisms while freezing the object throughout.

35. The method of claims 1 to 34, wherein said desired cooling effect is to promote the destruction and inactivation of surface microorganisms while cooling the object throughout.

36. A method of rapidly cooling objects comprising:
spraying said object with a fluid, and
spraying said object with an additive, wherein said sprayings are regulated to obtain a desired cooling effect.

37. The method of claim 36, wherein said additive is sprayed at one or more times as selected from the group consisting of prior to the spraying of the fluid, simultaneous with the spraying of the fluid, or subsequent to the spraying of the fluid.

38. A method of rapidly cooling objects comprising:
spraying said objects with a fluid,
spraying said objects with a first additive, and
spraying said objects with one or more additional additives, wherein said sprayings are regulated to obtain a desired cooling effect.

39. The method of claim 38, wherein said first additive and said one or more additional additives are different.

40. The method of claim 38, wherein said first additive and said one or more additional additives are the same.
